# EUROPEAN PATENT APPLICATION

(11) **EP 4 613 268 A1**
(43) Date of publication of application: **10.09.2025**
(21) Application number: 23885038.2
(22) Date of filing: 02.11.2023
(51) Int. Cl.: A61K 31/095, A61K 33/04, A61P 35/00

(54) **SELENIUM-CONTAINING SUBSTANCE FOR TREATMENT OF CANCER**

(30) Priority: 03.11.2022 CN 202211371380
(71) Applicant: Shanghai Institute of Nutrition and Health, Chinese Academy of Sciences, Shanghai 200031 (CN)
(72) Inventor: ZHAN, Lixing, Shanghai 200031 (CN); CAI, Peng, Shanghai 200031 (CN); YAO, Gang, Shanghai 200031 (CN); DING, Qiurong, Shanghai 200031 (CN); XU, Keying, Shanghai 200031 (CN); WEN, Qian, Shanghai 200031 (CN); XUE, Yuan, Shanghai 200031 (CN); JI, Weiwei, Shanghai 200031 (CN); JIAO, Jiazheng, Shanghai 200031 (CN); ZHANG, Jinlong, Shanghai 200031 (CN)
(74) Representative: dompatent von Kreisler Selting Werner - Partnerschaft von Patent- und Rechtsanwälten mbB
(86) International application number: PCT/CN2023/129292
(87) International publication number: WO 2024/094112

(57) **Abstract**

The present invention relates to a selenium-containing substance for the treatment of cancer. Specifically, the present invention provides use of a seleno-amino acid for preparing a composition or a preparation. The composition or the preparation is used for preventing and/or treating cancer. It is found for the first time in the present invention that the seleno-amino acid can effectively prevent and/or treat cancer.

## Description

### Technical field

The present invention relates to the biomedicine field. Specifically, it relates to a selenium-containing substance for the treatment of cancer.

### Background

Lung cancer is a cancer with the highest incidence and mortality rates globally, with a 5-year survival rate below 20%. It is classified into distinct histological subtypes, including adenocarcinoma, squamous cell carcinoma, and large cell carcinoma (commonly referred to as non-small cell lung cancer), as well as small cell lung cancer. NSCLC (LUAD) represents the most common histological subtype of non-small cell lung cancer (NSCLC), accounting for approximately 40% of lung malignancies. The primary etiologic risk factor for LUAD is smoking, while other reported risk factors mainly include long-term radon exposure, carcinogens, and air pollution, etc. In recent years, the incidence of non-smoking-related LUAD has been increasing annually. Despite advancements in cancer treatment in recent years, the overall survival rate for NSCLC patients remains low. Immunotherapy and chemotherapy are the two most widely used approaches for treating cancer. Although many cancer types initially respond to these treatments, the development of drug resistance is inevitable. The rapid development of drug resistance is a main characteristic of NSCLC. Therefore, there is an urgent need for the discovery of novel therapeutic agents.

Regulatory T cells (Tregs) are a type of T cells with prominent immunosuppressive functions, and they are a subset of T cells characterized by Foxp3, CD25, and CD4 cell phenotypes. Treg cells are known to exert negative regulatory effects on the body's immune response through intercellular contact with various immune cell subsets and secretion of inhibitory cytokines, thereby suppressing the body's immune responses and inducing active tolerance to self-antigens. However, in tumors, Treg cells, through their immunosuppressive effects, cause the body to develop antigenic tolerance to tumor cells. This allows tumor cells to undergo immune evasion, thereby enhancing their proliferation and infiltration capabilities, and enabling them to escape the body's immune killing effects. Thus, Treg cells are regarded as the type of immune cells that assist tumor survival and promote tumor growth. In recent years, reducing the function of Treg cells has become an important direction in tumor therapy.

Therefore, there is an urgent need in this field to develop effective selenium compounds with Treg cell-inhibiting properties, thereby achieving anti-tumor immunotherapy effects.

### Summary of the invention

The purpose of the present invention is to provide an effective selenium compound with Treg cell-inhibiting properties, thereby achieving anti-tumor immunotherapy effects.

Another purpose of the present invention is to provide an effective selenium-containing substance that effectively ameliorates tumor immune suppression characteristics and has an effective immunotherapy effect on non-small cell lung cancer.

In the first aspect of the present invention, is provided a use of a seleno-amino acid in the manufacture of a composition or formulation for preventing and/or treating a cancer.

In another preferred embodiment, the seleno-amino acid comprises a selenomethionine.

In another preferred embodiment, the seleno-amino acid comprises an L-seleno-amino acid.

In another preferred embodiment, the selenomethionine comprises an L-selenomethionine.

In another preferred embodiment, the cancer comprises lung cancer.

In another preferred embodiment, the lung cancer is selected from the group consisting of: adenocarcinoma, squamous cell carcinoma, large cell carcinoma (commonly referred to as non-small cell lung cancer), and a combination thereof.

In another preferred embodiment, the composition or formulation is also used for one or more uses selected from the group consisting of:
(a) inhibiting tumor growth;
(b) ameliorating the inhibitory state of tumor-suppressive immune cells;
(c) increasing the level of selenoproteins;
(d) reducing the number of infiltrating Treg cells in the tumor; and
(e) enhancing the immune response in the tumor microenvironment.

In another preferred embodiment, the selenoproteins comprise SOD1, SOD2, GPX1, GPX2, GPX4, Sephs2, Sepx1, SelS, SelP, Txnrd1, Txnrd2, and Txnrd3.

In another preferred embodiment, the tumor comprises lung cancer.

In another preferred embodiment, the composition comprises a pharmaceutical composition.

In another preferred embodiment, the pharmaceutical composition comprises (a) a seleno-amino acid; and (b) a pharmaceutically acceptable carrier.

In another preferred embodiment, the pharmaceutical composition is in a liquid, solid, or semi-solid form.

In another preferred embodiment, the dosage forms of the pharmaceutical composition comprise tablets, granules, capsules, oral liquids, or injections.

In another preferred embodiment, in the pharmaceutical composition, component (a) accounts for 1-99 wt%, preferably 10-90 wt%, and more preferably 30-70 wt% of the total weight of the pharmaceutical composition.

In another preferred embodiment, the composition further comprises other medicaments for preventing and/or treating a cancer.

In another preferred embodiment, the other medicaments for preventing and/or treating a cancer comprise PD-1 antibodies.

In another preferred embodiment, the composition or formulation can be used alone or in combination for applications in the prevention and/or treatment of a cancer.

In another preferred embodiment, the combined use comprises: using in combination with other medicaments for preventing and/or treating a cancer.

In the second aspect of the present invention, is provided a pharmaceutical composition, comprising:
(a1) a first active ingredient for preventing and/or treating a cancer, wherein the first active ingredient comprises: a seleno-amino acid;
(a2) optionally, a second active ingredient for preventing and/or treating a cancer, wherein the second active ingredient comprises: other medicaments for preventing and/or treating a cancer; and
(b) a pharmaceutically acceptable carrier.

In another preferred embodiment, in the pharmaceutical composition, the component (a1) accounts for 1-99 wt%, preferably 10-90 wt%, and more preferably 30-70 wt% of the total weight of the pharmaceutical composition.

In another preferred embodiment, in the pharmaceutical composition, the component (a2) accounts for 1-99 wt%, preferably 10-90 wt%, and more preferably 30-70 wt% of the total weight of the pharmaceutical composition.

In another preferred embodiment, the weight ratio of the first active ingredient to the second active ingredient is from 1:100 to 100:1, preferably from 1:10 to 10:1.

In another preferred embodiment, the other medicaments for preventing and/or treating a cancer comprise PD-1 antibodies.

In another preferred embodiment, the pharmaceutical composition may be a single compound or a mixture of multiple compounds.

In another preferred embodiment, the pharmaceutical composition is used for preparing a medicament or formulation for treating or preventing a cancer.

In another preferred embodiment, the dosage form of the medicament is an oral dosage form or a non-oral dosage form.

In another preferred embodiment, the oral dosage form is a tablet, powder, granule, capsule, emulsion or syrup.

In another preferred embodiment, the non-oral dosage form is an injection or a syringe.

In another preferred embodiment, the total content of the active ingredient (a1) and the active ingredient (a2) is 1-99 wt%, preferably 5-90 wt% of the total weight of the composition.

In the third aspect of the present invention, is provided a pharmaceutical kit comprising:
(i) a first container, and an active ingredient (a1) a seleno-amino acid, or a medicament comprising the active ingredient (a1), contained in the first container; and
(ii) optionally a second container, and an active ingredient (a2), other medicaments for preventing and/or treating a cancer, or a medicament comprising the active ingredient (a2), contained in the second container.

In another preferred embodiment, the first container and the second container are the same container or different containers.

In another preferred embodiment, the medicament in the first container is a single-ingredient preparation comprising the seleno-amino acid.

In another preferred embodiment, the medicament in the second container is a single-ingredient preparation comprising other medicaments for preventing and/or treating a cancer.

In another preferred embodiment, the dosage form of the medicament is an oral dosage form or an injectable dosage form.

In another preferred embodiment, the kit further comprises instructions, wherein the instructions specify the co-administration of the active ingredient (a1) and active ingredient (a2) for the prevention and/or treatment of a cancer.

In another preferred embodiment, the dosage forms of the preparation comprising the active ingredient (a1) a seleno-amino acid, or the active ingredient (a2) other medicaments for preventing and/or treating a cancer, respectively comprise capsules, tablets, suppositories, or intravenous injections.

In the fourth aspect of the present invention, is provided a use of the pharmaceutical composition of the second aspect of the present invention or the pharmaceutical kit of the third aspect of the present invention, in the manufacture of a medicament for preventing and/or treating a cancer.

In the fifth aspect of the present invention, is provided a method for preventing and/or treating a cancer, comprising a step of:
administering a seleno-amino acid, the pharmaceutical composition of the second aspect of the present invention or the pharmaceutical kit of the third aspect of the present invention, to a subject in need thereof.

In another preferred embodiment, the administration comprises oral administration.

In another preferred embodiment, the subject comprises humans or non-human mammals.

In another preferred embodiment, the non-human mammals comprise rodents and primates, preferably mice, rats, rabbits, and monkeys.

In another preferred embodiment, the administration frequency of the seleno-amino acid is 1-7 consecutive days per week, preferably 2-5 consecutive days per week, and more preferably 2-3 consecutive days per week.

In another preferred embodiment, the administration period of the L-seleno-amino acid is 1-20 weeks, preferably 2-12 weeks, and more preferably 4-8 weeks.

In the sixth aspect of the present invention, is provided a method for screening candidate medicaments for preventing and/or treating a cancer, comprising the steps of:
(a) in a test group, in the presence of a test substance, culturing cells expressing selenoproteins for a period of time T1 in a culture system, and detecting the expression level E1 and/or activity A1 of selenoproteins in the culture system of the test group;
and in a control group where the test substance is absent and other conditions are the same, detecting the expression level E2 and/or activity A2 of selenoproteins in the culture system of the control group;
(b1) comparing E1 and E2, and if E1 is significantly higher than E2, it indicates that the test substance is a candidate medicament for preventing and/or treating a cancer; and/or
(b2) comparing A1 and A2, and if A1 is significantly higher than A2, it indicates that the test substance is a candidate medicament for preventing and/or treating a cancer.

In another preferred embodiment, the selenoproteins comprise SOD1, SOD2, GPX1, GPX2, GPX4, Sephs2, Sepx1, SelS, SelP, Txnrd1, Txnrd2, and Txnrd3.

In another preferred embodiment, the cells are mammalian cells.

In another preferred embodiment, the cells comprise tumor cells.

In another preferred embodiment, the cells are selected from the group consisting of: lung cancer cells, liver cancer cells, breast cancer cells, and combinations thereof.

In another preferred embodiment, the cells are selected from the group consisting of: LLC, HTB-182, CRL-5803, A549, and combinations thereof.

In another preferred embodiment, the cells are cells cultured *in vitro.*

In another preferred embodiment, the "significantly higher than" means E1/E2 ≥ 2, preferably ≥ 3, and more preferably ≥ 4.

In another preferred embodiment, the "significantly higher than" means A1/A2 ≥ 2, preferably ≥ 3, and more preferably ≥ 4.

In another preferred embodiment, the method is non-diagnostic and non-therapeutic.

In another preferred embodiment, the method comprises step (c): administering the candidate medicament identified in step (a) to a non-human mammal, thereby determining its effect on cancer in the non-human mammal.

In another preferred embodiment, the test substance is selected from the group consisting of: small molecule compounds, antibodies, polypeptides, nucleic acids, and combinations thereof.

It should be understood that within the scope of the present invention, the above-mentioned technical features of the present invention and the technical features specifically described in the following (such as the examples) can be combined with each other to form a new or preferred technical solution, which is not redundantly repeated one by one herein due to space limitation.

### Description of the drawings

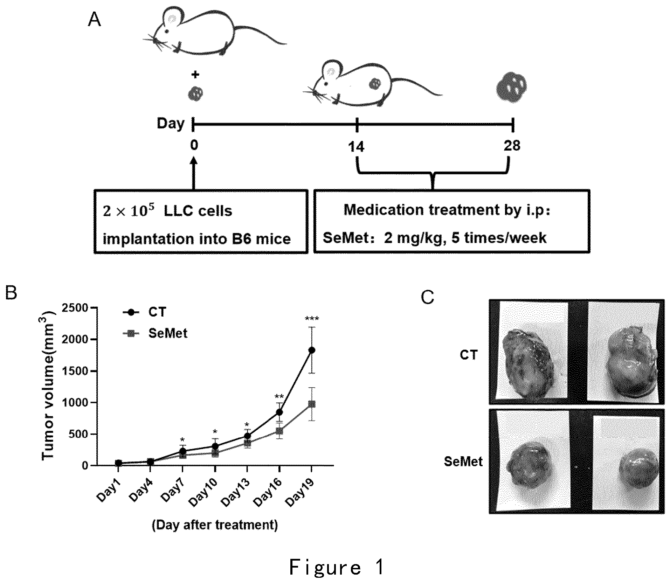

Figure 1 demonstrates that SeMet has good therapeutic effects on LLC cell subcutaneous xenograft tumors in mice. Figure 1A: Experimental pattern diagram. Mice were first subcutaneously injected with LLC cells (2*10^5 cells per mouse) to establish xenograft tumors. After 14 days of tumor formation, mice received SeMet treatment for two weeks, with intraperitoneal injection of SeMet at a dose of 2 mg/kg, 5 times per week; Figure 1B: Tumor growth statistical curve in mice; Figure 1C: Representative photographic results of mouse tumors.

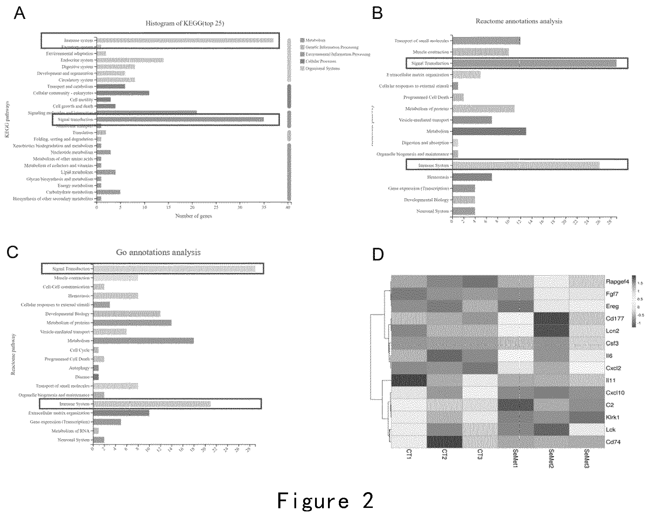

Figure 2 illustrates the RNA-Seq sequencing results of LLC xenograft tumors after SeMet treatment. Figure 2A-C: Alignment with KEGG/Reactome/GO databases revealed significant changes in signaling transduction and immune system following selenium treatment. Figure 2D: A functional enrichment chord diagram obtained from the RNA-Seq sequencing of LLC xenograft tumors after SeMet treatment, which indicates significant changes in immune positive regulation and cytokine stimulation after SeMet treatment. This suggests a positive change in the tumor immune microenvironment after selenium treatment.

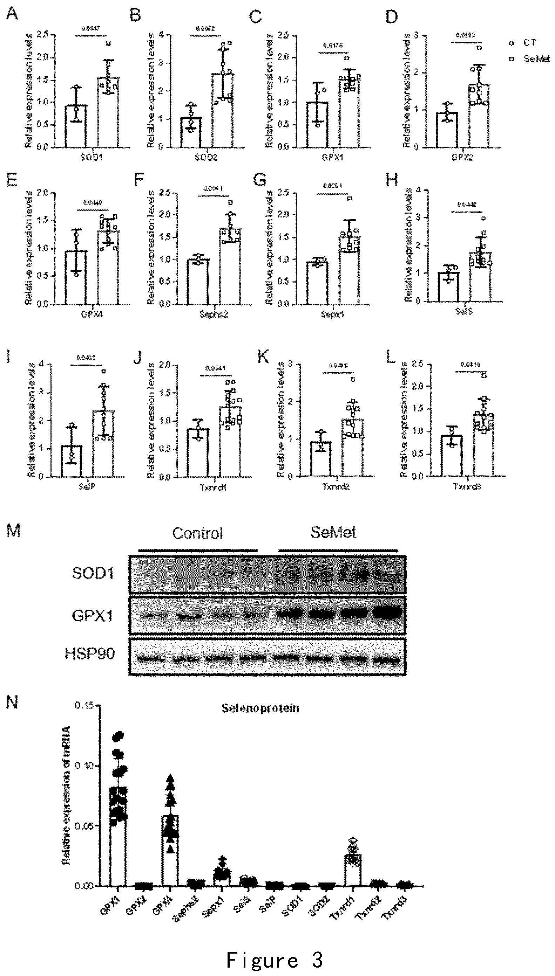

Figure 3 demonstrates that SeMet treatment enhances selenoprotein expression in LLC xenograft tumors, validating selenium supplementation efficacy. Figure 3A-L: qPCR analysis of the expression of selenoproteins SOD1, SOD2, GPX1, GPX2, GPX4, Sephs2, Sepx1, SelS, SelP, Txnrd1, Txnrd2, and Txnrd3 in xenograft tumors from control and SeMet-treated groups at the mRNA level; Figure 3M: Western blot (WB) analysis of SOD1 and GPX1 protein expression in xenograft tumors from control and SeMet-treated groups; Figure 3M: qPCR analysis of relative expression levels of various selenoprotein genes in subcutaneous tumors after SeMet treatment.

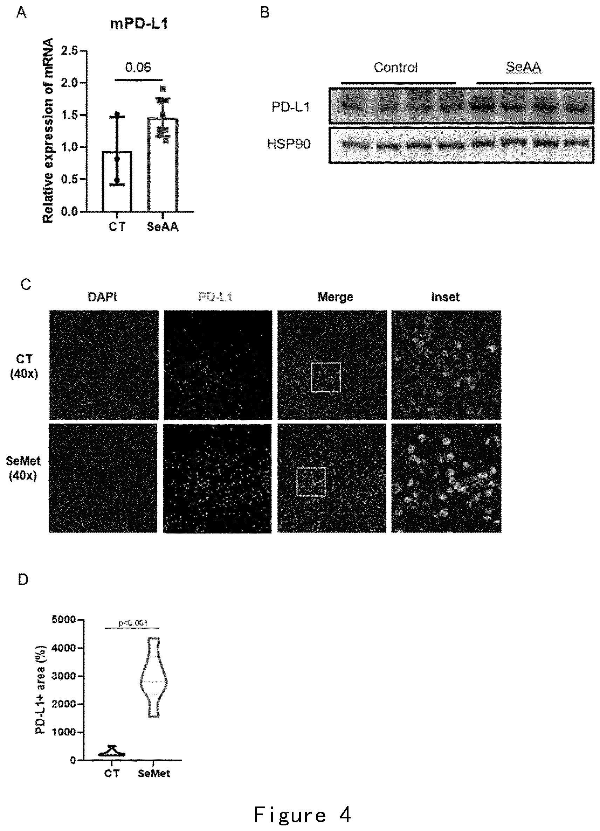

Figure 4 demonstrates that SeMet treatment enhances PD-L1 expression in LLC xenograft tumors. Figure 4A: qPCR analysis of PD-L1 expression in xenograft tumors from control and SeMet-treated groups at the mRNA level; Figure 4B: WB analysis of PD-L1 protein expression in xenograft tumors from control and SeMet-treated groups; Figure 4C: Immunofluorescence analysis of PD-L1 protein expression in subcutaneous tumors from control and SeMet-treated groups; Figure 4D: Quantitative statistical chart of Figure 4C.

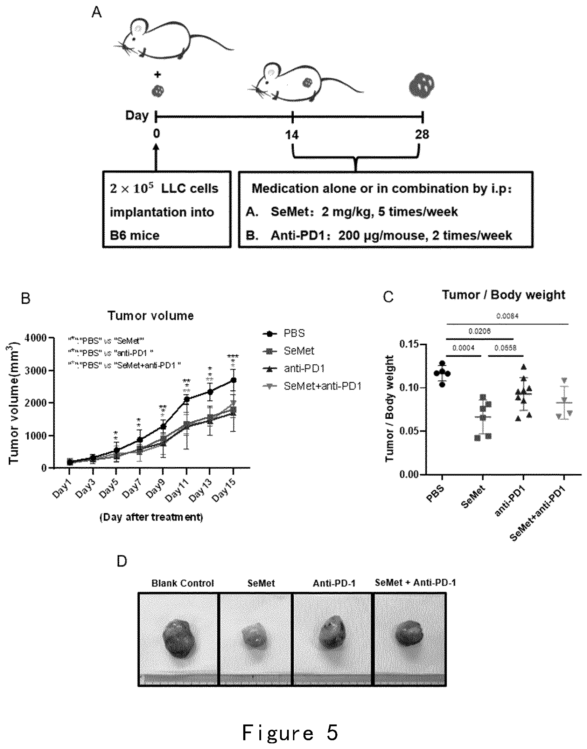

Figure 5 illustrates the therapeutic effects of SeMet and PD-1 antibody on LLC cell subcutaneous xenograft tumors in mice. Figure 5A: Experimental pattern diagram. Mice were first subcutaneously injected with LLC cells (2*10^5 cells per mouse) to establish xenograft tumors. After 14 days of tumor formation, mice were treated with SeMet and PD-1 antibody alone or in combination by intraperitoneal injection for two weeks. The dosage of SeMet was 2 mg/kg, 5 times per week; the dosage of PD-1 antibody was 200 µg per mouse, 2 times per week; Figure 5B: Tumor growth statistical curve in mice; Figure 5C: Ratio of tumor to body weight in mice; Figure 5D: Representative photographic results of mouse tumors.

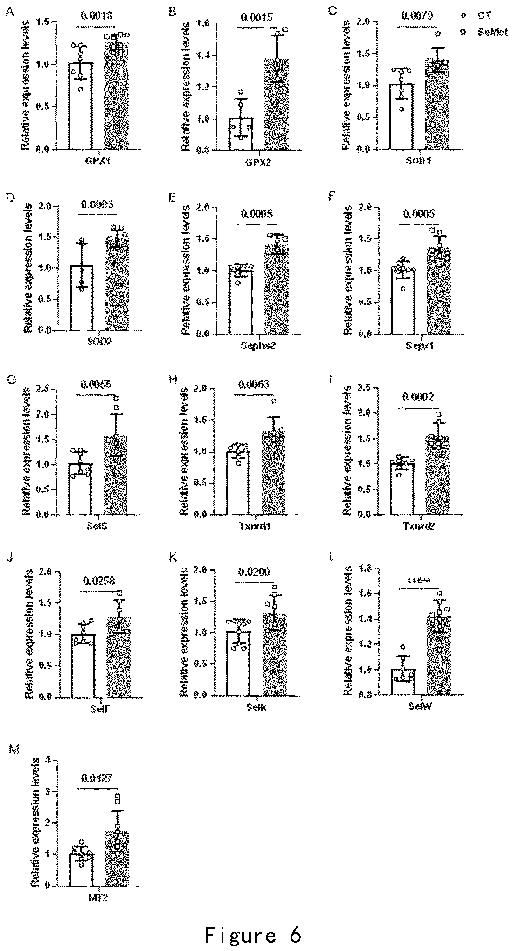

Figure 6 demonstrates that SeMet treatment enhances selenoprotein expression in LLC xenograft tumors. Figure 6A-M: qPCR analysis of expression of selenoproteins GPX1, GPX2, SOD1, SOD2, Sephs2, Sepx1, SelS, Txnrd1, Txnrd2, SelF, Selk, SelW, and MT2 in xenograft tumors from control and SeMet-treated groups at the mRNA level.

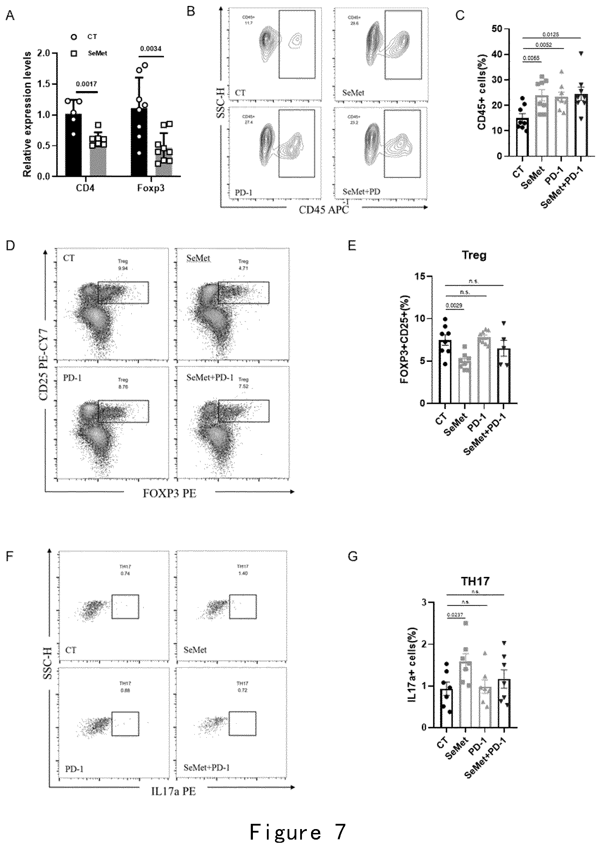

Figure 7 demonstrates that SeMet treatment inhibits the expression of Treg cells in LLC xenograft tumors. Figure 7A: qPCR analysis of CD4 and Foxp3 expression in xenograft tumors from control and SeMet-treated groups at the mRNA level. Figure 7B: Flow cytometry analysis of the percentage of CD45+ immune cells in xenograft tumors from control and SeMet-treated groups. Figure 7C: Quantitative statistical result chart of the percentage of CD45+ immune cells in Figure 7B. Figure 7D: Flow cytometry analysis of the percentage of CD25+FOXP3+ cells in xenograft tumors from control and SeMet-treated groups. Figure 7E: Quantitative statistical result chart of the percentage in Figure 7D. Figure 7F: Flow cytometry analysis of the percentage of IL17a+ cells in xenograft tumors from control and SeMet-treated groups. Figure 7G: Quantitative statistical result chart of the percentage in Figure 7F.

### Detailed description

After extensive and in-depth research, the inventors unexpectedly discovered for the first time that a seleno-amino acid (such as L-seleno-amino acid, preferably L-selenomethionine) can significantly inhibit tumor growth and effectively prevent and/or treat a cancer.

Specifically, in the present invention, it is first found that in the NSCLC subcutaneous xenograft tumor model, the supplementation of L-SeMet can significantly inhibit tumor growth. By comparing its therapeutic effect with that of a specific PD-1 antibody, the results show that its effect in this model is similar to that of PD-1 therapy. More specifically, compared with the control group, after L-SeMet supplementation, both the tumor volume and tumor mass were significantly reduced. The specific data showed no significant difference from those of the PD-1 group, indicating a similar effect. Based on this, the inventors further conducted RNA-seq detection. The specific data analysis showed that after L-SeMet treatment, by comparing with the three major databases of KEGG/Reactome/GO, significant changes occurred in the tumor immune system. The main immune mechanisms by which L-SeMet treatment ameliorates lung cancer may include: the positive regulation of immunosuppressive signals and the regulation of key immune pathways, specifically through promoting TH17 differentiation and exerting immunomodulatory effects on inhibitory Treg cells. The functional enrichment chord diagram obtained from the analysis of these results indicates that there are significant changes in positive immune regulation and cytokine stimulation after selenium treatment, suggesting that the tumor immune microenvironment has been positively altered after selenium treatment.

In addition, the applicant further extracted NSCLC tissues and found that after L-SeMet treatment, the levels of major selenoproteins, including GPX1, GPX4 and other main selenoproteins, were significantly increased. The applicant further extracted relevant treated tissues, extracted immune cells according to conventional methods and performed flow sorting, which confirmed that the number of Treg cells infiltrating the tumor was significantly reduced after L-SeMet treatment. The tumor-infiltrating Treg population often plays a key role in tumor immune evasion by inhibiting the function of immune cells attacking the tumor. This confirms that the reduction in the number of Treg cells promotes the enhancement of the tumor immune response, thereby significantly preventing the progression of subcutaneous NSCLC. Therefore, L-SeMet can be used as an effective organic substance to inhibit NSCLC growth by significantly ameliorating the inhibitory state of tumor-suppressive immune cells. On this basis, the inventors completed the present invention.

### Lung cancer

Lung cancer is the cancer with the highest incidence and mortality rates globally, with a 5-year survival rate below 20%. It is classified into distinct histological subtypes, including adenocarcinoma, squamous cell carcinoma, and large cell carcinoma (commonly referred to as non-small cell lung cancer), as well as small cell lung cancer. NSCLC (LUAD) represents the most common histological subtype of non-small cell lung cancer (NSCLC), accounting for approximately 40% of lung malignancies. The primary etiologic risk factor for LUAD is smoking, while other reported risk factors mainly include long-term radon exposure, carcinogens, and air pollution, etc. In recent years, the incidence of non-smoking-related LUAD has been increasing annually. Despite advancements in cancer treatment in recent years, the overall survival rate for NSCLC patients remains low. Immunotherapy and chemotherapy are the two most widely used approaches for treating cancer. Although many cancer types initially respond to these treatments, the development of drug resistance is inevitable. The rapid development of drug resistance is a main characteristic of NSCLC. Therefore, there is an urgent need for the discovery of novel therapeutic agents.

### Seleno-amino acid

Selenium is an important essential trace element for the human body. It is known that selenium can inhibit tumor growth through various pathways, such as improving immune function, enhancing the body's anti-tumor oxidation ability, and blocking tumor angiogenesis, via selenoproteins. The special metabolic products of selenium can change the tumor immune microenvironment through different pathways, thereby inhibiting the further development of tumors. Selenium can enhance the functions of various immune cells, such as dendritic cells, T lymphocytes, and NK cells. However, while selenium supplementation, as a method to improve population immunity, is widely used to enhance immunity in the general population, there has been a lack of clear evidence regarding the specific mechanism of its immunotherapeutic efficacy in cancer patients over the years. Some researchers have found that high-selenium intake can enhance the activation and function of T cells and B cells, and has a positive effect on the proliferation and differentiation of the Th4 cell cluster, so as to enhance the immune effect of virus antigen vaccines. At the same time, other researchers have explored and found that in human and rodent models, the expression of selenoproteins is often positively correlated with the survival of HCC patients and is related to immune response pathways such as M1 macrophage polarization.

In the present invention, the seleno-amino acid refers to a selenium-containing amino acid in which the S in the methionine molecule is replaced by Se, mainly including the L-seleno-amino acid.

In the present invention, the L-seleno-amino acid comprises the L-selenomethionine.

In the present invention, it is discovered for the first time that L-seleno-amino acids can significantly inhibit tumor growth and can effectively prevent and/or treat a cancer.

### Pharmaceutical composition and administration method

In another aspect, the present invention also provides a pharmaceutical composition, comprising (a) a safe and effective amount of the seleno-amino acid of the present invention (such as L-seleno-amino acid); and (b) a pharmaceutically acceptable carrier or excipient. The dosage of the seleno-amino acid of the present invention (such as L-seleno-amino acid) is usually 10 micrograms-100 milligrams per dose, preferably 100-1000 micrograms per dose. For the purposes of the present invention, an effective dosage administered to an individual is about 0.01 milligrams per kilogram of body weight to 50 milligrams per kilogram of body weight, preferably 0.05 milligrams per kilogram of body weight to 10 milligrams per kilogram of body weight of the seleno-amino acid of the present invention (such as L-seleno-amino acid). In addition, the seleno-amino acid of the present invention (such as L-seleno-amino acid) can be used alone or in combination with other therapeutic agents (e.g., formulated in the same pharmaceutical composition).

The pharmaceutical composition may also comprise a pharmaceutically acceptable carrier. The term "pharmaceutically acceptable carrier" refers to a carrier used for the administration of a therapeutic agent. This term refers to those pharmaceutical carriers that themselves do not induce the production of antibodies harmful to the individual receiving the composition and do not cause excessive toxicity after administration. These carriers are well-known to those of ordinary skill in the art. A full discussion of pharmaceutically acceptable excipients can be found in Remington's Pharmaceutical Sciences (Mack Pub. Co., N.J. 1991). Such carriers include (but are not limited to): saline, buffers, glucose, water, glycerol, ethanol, adjuvants, and combinations thereof.

The pharmaceutically acceptable carrier in the therapeutic composition may comprise liquids such as water, saline, glycerol, and ethanol. In addition, auxiliary substances, such as wetting agents or emulsifiers, pH-buffering substances, etc., may also be present in these carriers.

Generally, the therapeutic composition can be prepared as an injection, such as a liquid solution or suspension; it can also be prepared in a solid form suitable for incorporation into a solution or suspension or a liquid carrier before injection.

Once the composition of the present invention is prepared, it can be administered by conventional routes, including (but not limited to): intratumoral, intramuscular, intravenous, subcutaneous, intradermal, or topical administration. The subject to be prevented or treated can be animals; especially humans.

When the pharmaceutical composition of the present invention is used for actual treatment, various dosage forms of the pharmaceutical composition can be adopted according to the usage situation. Preferably, the dosage form is an intravenous administration formulation or an intratumoral administration injection.

These pharmaceutical compositions can be formulated by mixing, diluting, or dissolving according to conventional methods, and occasionally appropriate pharmaceutical additives such as excipients, disintegrants, binders, lubricants, diluents, buffers, isotonicities, preservatives, wetting agents, emulsifiers, dispersants, stabilizers, and solubilizers are added, and the formulation process can be carried out in a customary manner according to the dosage form.

For example, the formulation of eye drops can be carried out as follows: dissolving the seleno-amino acid of the present invention (such as L-seleno-amino acid) together with the basic substances in sterile water (in which a surfactant is dissolved), adjusting the osmotic pressure and pH to the physiological state, and optionally adding appropriate pharmaceutical additives such as preservatives, stabilizers, buffers, isotonic agents, antioxidants, and thickeners, and then completely dissolving them.

The pharmaceutical composition of the present invention can also be administered in the form of a sustained-release agent. For example, the seleno-amino acid of the present invention (such as L-seleno- amino acid) can be incorporated into pills or microcapsules with a sustained-release polymer as a carrier, and then the pills or microcapsules are surgically implanted into the tissue to be treated. As an example of sustained-release polymers, ethylene-vinyl acetate copolymer, polyhydrometaacrylate, polyacrylamide, polyvinylpyrrolidone, methylcellulose, lactic acid polymer, and lactic acid-glycolic acid copolymer, etc., can be cited. Preferably, a biodegradable polymer, such as lactic acid polymer and lactic acid-glycolic acid copolymer can be cited.

When the pharmaceutical composition of the present invention is used for actual treatment, the dosage of the seleno-amino acid of the present invention (such as L-seleno-amino acid) as the active ingredient can be reasonably determined according to the body weight, age, gender, and symptom severity of each patient to be treated.

### Main advantages of the present invention include:

(1) The present invention discovered for the first time that seleno-amino acids (e.g., L-seleno-amino acids, preferably L-selenomethionines) can significantly inhibit tumor growth and effectively prevent and/or treat a cancer.
(2) In the present invention, it is discovered for the first time that the supplementation of L-SeMet can significantly inhibit tumor growth. By comparing its therapeutic effect with that of a specific PD-1 antibody, the results show that its effect in this model is similar to that of PD-1 therapy. More specifically, compared with the control group, after L-SeMet supplementation, both the tumor volume and tumor mass were significantly reduced. The specific data showed no significant difference from those of the PD-1 group, indicating a similar effect. Based on this, the inventors further conducted RNA-seq detection. The specific data analysis showed that after L-SeMet treatment, by comparing with the three major databases of KEGG/Reactome/GO, significant changes occurred in the tumor immune system. The main immune mechanisms by which L-SeMet treatment ameliorates lung cancer may include: the positive regulation of immunosuppressive signals and the regulation of key immune pathways, specifically through promoting TH17 differentiation and exerting immunomodulatory effects on inhibitory Treg cells. The functional enrichment chord diagram obtained from the analysis of these results indicates that there are significant changes in positive immune regulation and cytokine stimulation after selenium treatment, suggesting that the tumor immune microenvironment has been positively altered after selenium treatment.
(3) In the present invention, it is discovered for the first time that the levels of major selenoproteins, including GPX1, GPX4 and other main selenoproteins, were significantly increased. The applicant further extracted relevant treated tissues, extracted immune cells according to conventional methods and performed flow sorting, which confirmed that the number of Treg cells infiltrating the tumor was significantly reduced after L-SeMet treatment. The tumor-infiltrating Treg population often plays a key role in tumor immune evasion by inhibiting the function of immune cells attacking the tumor. This confirms that the reduction in the number of Treg cells promotes the enhancement of the tumor immune response, thereby significantly preventing the progression of subcutaneous NSCLC. Therefore, L-SeMet can be used as an effective organic substance to inhibit NSCLC growth by significantly ameliorating the inhibitory state of tumor-suppressive immune cells.

The present invention will be further illustrated below with reference to the specific examples. It should be understood that these examples are only to illustrate the present invention, not to limit the scope of the present invention. The conditions of the experimental methods not specifically indicated in the following examples are usually in accordance with conventional conditions as described in e.g., Sambrook et al., Molecular Cloning: A Laboratory Manual (New York: Cold Spring Harbor Laboratory Press, 1989), or according to the conditions recommended by the manufacturers. Percentages and parts are calculated by weight unless otherwise stated.

Unless otherwise specified, the materials and reagents used in the examples of the present invention, are commercially available products.

### Materials and methods

### 1.1 Experimental materials

### 1.1.1 Cell lines

The NSCLC cell line LLC was purchased from the Cell Bank of the Chinese Academy of Sciences.

### 1.1.2 Reagents and consumables

DMEM medium and RPMI-1640 medium were purchased from Thermo Fisher Scientific Inc.; fetal bovine serum (FBS) was purchased from BI; trypsin-EDTA and penicillin-streptomycin were both purchased from Gibco; goat serum was purchased from Life Technology. Various centrifuge tubes, cryopreservation tubes, culture dishes and pipettes used for cell culture were purchased from Corning Incorporated. 0.22 µm and 70 µm filters were purchased from Millipore Corporation; imported eppendorf tubes and pipette tips were purchased from Axygen. Paraformaldehyde, dimethyl sulfoxide (DMSO), polybrene, puromycin, pepstatin, PMSF, aprotinin, vanadate, leupeptin, diethyl pyrocarbonate (DEPC), ammonium persulfate (APS), and tetramethylethylenediamine (TEMED), etc., were all purchased from Sigma-Aldrich. TRIzol RNA extraction reagent was purchased from Invitrogen Corporation. RT-qPCR kit and PrimerSTAR Max DNA Polymerase kit were purchased from TaKaRa Bio Inc. The fluorescein quantification reagent SYRB Premix Ex TaqTM was from Yeasen Biotechnology Co., Ltd. The protein quantification kit - BCA assay kit was purchased from Thermo Fisher Scientific Inc. PVDF membrane and ECL chemiluminescence kit used for Western blotting were purchased from Millipore Corporation. Reagents, such as absolute ethanol, isopropanol, methanol, chloroform, sodium dodecyl sulfate (SDS), glycerol, trihydroxymethyl aminomethane (Tris), glycine, mercaptoethanol, bovine serum albumin (BSA), Tween-20, potassium chloride, sodium chloride, sodium azide, and bromophenol blue, etc., were all purchased from Shanghai Dingguo Biotechnology Co., Ltd. 30% polyacrylamide and 10×PBS phosphate buffer were both purchased from Sangon Biotech Co., Ltd. All primers were synthesized by Shanghai Generay Biotech Co., Ltd. The tumor cell isolation kit was purchased from Miltenyi Biotec, and CD45, CD3, CD4, CD25, Foxp3, IL-17A antibodies were purchased from Invitrogen Corporation. The fixation/permeabilization reagent was purchased from Becton, Dickinson and Company.

### 1.1.3 Main experimental instruments

Phase-contrast microscope, 37°C CO₂ cell incubator (Thermo Fisher), clean bench (Heraus), pipette (Eppendorf), pipettor (Thermo Fisher), electro-thermal constant temperature blast drying oven (Hengke), constant temperature water bath (Jinghong), metal bath (Weixing), horizontal shaker (Kylin-bell), vortex oscillator (Kylin-bell), temperature-controlled shaker (Peiying), protein electrophoresis apparatus (Bio-Rad), high-speed desktop low-temperature centrifuge (Eppendorf), low-temperature refrigerator, ultra-low-temperature refrigerator, scanner (Hewlett-Packard), chemiluminescence imager (BioRad), upright/inverted integrated fluorescence microscope (ECHO), Olympus laser scanning confocal microscope, automatic sample rapid grinder (Shanghai Jingxin Technology), fluorescent quantitative PCR instrument QuantStudio 6 (Thermo Fisher), boiling apparatus for protein samples (Weixing), ultra-micro nucleic acid and protein analyzer Nano Drop (Thermo Fisher Scientific), analytical flow cytometer CytoFlex LX (Beckman Coulter).

### 1.2 Experimental methods

### 1.2.1 Cell culture and subculture

The mouse lung cancer cell line LLC was cultured in DMEM (containing 10% FBS and 1% penicillin-streptomycin). Using a 10 cm dish as an example, cells were passaged at a 1:3 ratio for cell subculture. When the cells grew to over 90%, the culture medium was discarded, and cells were washed once with PBS. Then, 1 mL of trypsin was added for digestion for several minutes. When the cells completely rounded up, 2 mL of complete medium was added to quench the digestion. The cells were removed from the dish, transferred to a 15 mL centrifuge tube, centrifuged at 900 rpm for 3 minutes. After centrifugation, the supernatant was discarded. The cells were resuspended in 1 mL of culture medium, and 330 µL of the suspension was transferred to a new 10 cm dish. Subsequently, 10 mL of DMEM culture medium was added and mixed well. All cultures were performed in an incubator at 37°C with 5% CO₂.

### 1.2.2 Protein extraction and Western blotting

First, a protein lysis buffer containing protease inhibitors was prepared (the protease inhibitors, which were respectively 1000× Aprotinin, 1000× Leupeptin, 1000× Pepstain A, 1000× Sodium ortho vanadate, and 100× PMSF, were added in proportion to the RIPA lysis). After cell culture or treatment, the culture medium was carefully discarded, and the cells were washed once with ice-cold PBS. Then, a certain volume of protein lysis buffer was added, and the cells were scraped off using a cell scraper. For tissue protein extraction, 20-30 mg of tissue was obtained, and added with steel beads and 1 ml of protein lysis buffer. The mixture was then ground into a protein homogenate at 60 Hz for 2 minutes. The lysis buffer was placed on ice for 30 minutes and then centrifuged at 13,200 rpm for 15 minutes at 4°C. Subsequently, the protein supernatant was transferred to a clean eppendorf tube. At this point, a BCA reagent was prepared (A solution: B solution = 1:50). In a 96-well plate, 2 µL of protein samples was added in triplicate for each sample, along with 0, 2, 4, 6, 8, and 10 µL of 2 mg/ml standard solutions. Then, 200 µL of the BCA reagent was added to each well, and the plate was incubated at 37°C for 30 minutes. The OD values were detected at 562 nm using a microplate reader. The protein concentrations were calculated, and the protein samples were diluted to a uniform concentration with the RIPA lysis. Finally, the protein samples were added with 5× loading buffer (containing β-mercaptoethanol), boiled at 100°C for 10 minutes, and stored at -20°C.

A SDS-PAGE protein gel of a certain concentration, 1× running buffer, and 1× transfer buffer (pre-cooled at 4°C) were prepared. Protein samples were thawed and mixed well at room temperature. The prepared SDS-PAGE protein gel was fixed onto the electrophoresis apparatus, 1× running buffer was added, 20-30 µg of protein was loaded per well, and then the power supply was connected. Electrophoresis was carried out at 80 V until the protein samples migrated into the separating gel, then the voltage was adjusted to 120 V and maintained until the protein samples reached the bottom of the gel. Once the bromophenol blue migrated out, the gel was removed. The pre-cut PVDF membrane was activated with methanol. The transfer clamp was soaked in transfer buffer, then opened with the black side facing down and the white side facing up. The stacking gel part of the protein gel was cut off with tools, and the protein gel was carefully removed in the transfer buffer, then arranged and assembled according to the order of black side-filter paper-protein gel-PVDF membrane-filter paper-white side. The transfer clamp was closed, ensuring that any air bubbles between the gel and PVDF membrane were expelled. The transfer clamp was placed in the electrophoresis tank, and an appropriate amount of transfer buffer and ice packs were added. The membrane was transferred at a constant current of 240 mA for 120-150 minutes. After the membrane transfer, the protein was transferred from the gel to the PVDF membrane. The PVDF membrane was removed with the side facing the gel upward, which was considered the front side. The membrane protein bands were cut according to the molecular weight of the desired protein, blocked with 5% skim milk for 1-2 hours at room temperature, and then washed several times with TBST after blocking. The primary antibody diluted in 3% BSA was added, and the bands were incubated overnight at 4°C. The next day, the primary antibody was recovered, and the bands were washed three times with TBST for 10 minutes each. The secondary antibody diluted in 5% milk was added, and the bands were incubated for 1-2 hours at room temperature, and then washed three times with TBST for 10 minutes each. Finally, the bands were developed using ECL luminescent liquid.

### 1.2.3 Immunofluorescence

Paraffin-embedded tissue sections were obtained, incubated in a 65°C oven for 30 minutes to 1 h, incubated in xylene for two times (each 10 minute) for dewaxing. Afterwards, hydration was conducted through sequential incubations in 100% ethanol, 95% ethanol, 80% ethanol, and 70% ethanol for 5 minutes each. The sections were then washed twice with ddH₂O for 5 minutes each. Subsequently, the sections were treated with a methanol solution containing 3% hydrogen peroxide in darkness for 15 minutes, to inactivate the peroxidase activity within the tissue. The sections were then washed three times with PBS for 5 minutes each, and retrieved in a pressure cooker using 0.01M sodium citrate buffer (pH 6.0) for 2 minutes. Then, the sections were allowed to cool and depressurize gradually, followed by three 5-minute washes with PBST. Afterwards, the sections were blocked with a PBS solution containing 10% goat serum and 0.01% Triton X-100 for 1 hour at room temperature. After blocking, the primary antibody was diluted with the blocking solution according to the manufacturer's instructions of the antibody, and the sections were incubated with the primary antibody overnight at 4°C. The next day, the sections were washed three times with PBST for 10 minutes each.

The fluorescent secondary antibody was diluted at a ratio of 1:200, and the sections were incubated with the fluorescent secondary antibody for 1 hour at room temperature in darkness. After three 10-minute washes with PBST, the sections were stained with DAPI for 15 minutes and washed three times with PBST for 5 minutes each. Finally, the sections were mounted with a coverslip.

### 1.2.4 RNA extraction, reverse transcription, and real-time PCR

After the cells were cultured or treated, the medium was discarded, and the cells were washed once with ice-cold PBS. Then, 1 mL of Trizol reagent was added, and the cells were completely blown down. If it was for the tissue, 10-20 mg of tissue was homogenized into a homogenate using a grinder, with the addition of steel beads and 1 mL of Trizol. 200 µL of chloroform was added into the cell or tissue homogenate in 1 mL of Trizol, mixed vigorously, and placed on ice for 5 minutes. The mixture was centrifuged at 12,000 rpm for 10 minutes at 4°C. Then, 400 µL of the supernatant was carefully aspirated and added to an equal volume of isopropanol, vigorously mixed, and placed on ice for 15 minutes and centrifuged again at 12,000 rpm for 10 minutes at 4°C. Afterwards, a white RNA precipitate was visible at the bottom. The supernatant was carefully discarded, and 1 ml of 75% ethanol (diluted with DEPC-treated water) was added. The tube was flicked gently to resuspend the white precipitate, followed by centrifugation at 8,000 rpm for 5 minutes at 4°C, and these steps were repeated once. After the final centrifugation, all supernatant was removed, and the precipitate was air-dried at room temperature until semitransparent. A certain volume of DEPC-treated water was added to dissolve the RNA, and the concentration was measured. RNA reverse transcription included two parts: genomic DNA removal and reverse transcription. The genomic DNA removal reaction system was as follows:

| Reagent | Volume |
|---|---|
| 5× gDNA Eraser Buffer | 2 µL |
| gDNA Eraser | 1 µL |
| Total RNA | 1 µg |
| DEPC-treated water | Up to 10 µL |
| Total | 10 µL |

42°C 2 min. 4°C hold.

The reverse transcription reaction system was as follows:

| Reagent | Volume |
|---|---|
| Genomic DNA removal reaction mixture | 10 µL |
| Primer Script RT Enzyme Mix I | 1 µL |
| RT Enzyme Mix | 4 µL |
| 5× Primer Script Buffer2 (for real time) | 4 µL |
| DEPC-treated water | 1 µL |
| Total | 20 µL |

37°C, 30 min. 85°C 5 s. 4°C hold.

The reverse-transcribed cDNA was diluted 25-fold with sterile distilled water, mixed thoroughly, and stored at -20°C as the template for the real time reaction.

The real time reaction system was as follows:

| Reagent | Volume |
|---|---|
| 2× SYRB Green buffer | 5 µL |
| Primer 1 (10 µM) | 0.2 µL |
| Primer 2 (10 µM) | 0.2 µL |
| Sterile water | 0.6 µL |
| Diluted cDNA template | 4 µL |
| Total | 10 µL |

According to the gene primers to be detected, the above-mentioned Mix without the cDNA template was prepared, mixed thoroughly, and kept on ice until use. The 384-well plate was prepared and samples were distributed as needed: first, cDNA templates from different samples were added to the wells, followed by the mixed primers and the SYBR Green Mix. Notice: contamination during pipetting shall be avoided. The 384-well plate was centrifuged at 2,000 rpm for 2 minutes at room temperature to spin down any residual liquid on the well walls, ensuring no liquid remained adhering to the walls. Subsequently, the detection program and sample loading configuration were set up, and the QuantStudio^{™} 6 Flex System was used for detection. The detection program was as follows:

| | |
|---|---|
| 95°C 5 min | |
| 95 °C 10 s | 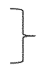 30 cycles |
| 60 °C 30 s | |
| 95 °C 15 s | 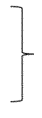 Melt Curve Stage |
| 60 °C 1 min | |
| 95 °C 15 s | |
| End | |

After detection, the relative gene expression levels were calculated using the 2^{-▲▲}T method.

### 1.2.5 Flow cytometry

Preparation of single-cell suspension from mouse tumor cells: 200 mg of mouse tumor tissue was obtained, cut into pieces and added to 2.43 ml of tumor digestion solution, followed by digestion in a 37°C shaking water bath for 30 minutes. Flow cytometry staining included surface antigen staining and intracellular factor (cytokines and transcription factors) staining. For the cytokine detection, broad-spectrum activation inhibitors (PMA, ionomycin, or BFA, added at a ratio of 1:1000) were first prepared with the RPMI-1640 medium. 100 µl of each reagent was added to resuspend the cells, and the cells were then transferred to a 96-well plate and treated in a 37°C cell incubator for 4-6 h. Subsequently, 200 µl of FACS buffer was added, and the cells were pipetted to mix evenly before transferred to a 1.5 ml eppendorf tube. The tube was centrifuged at 350 g for 5 minutes, and the supernatant was carefully aspirated. Cells were resuspended in 100 µl of PBS. For the surface antigen staining, the above steps were omitted. The cell suspension was placed on ice, and live/dead dye (at a ratio of 1:1000) was added. Staining was performed on ice in darkness for 30 minutes. Cells were washed with 1 ml of PBS, centrifuged at 350 g for 5 minutes, and the supernatant was carefully removed. The cell precipitate was resuspended in the FACS buffer, and the blocking antibody CD16/CD32 was added at a ratio of 1:200. The mixture was placed on ice in darkness for 30 minutes. Subsequently, surface antibodies were added at a ratio of 1:200: CD45, CD3, CD4, and CD25 antibodies were sequentially added for Treg cell surface staining; CD45, CD3, and CD4 antibodies were sequentially added for Th17 cell staining. Incubation was performed on ice in darkness for 30 minutes. Cells were washed with 1 ml of the FACS buffer, then centrifuged at 350 g for 5 minutes, and the supernatant was carefully removed. For intracellular factor staining, 250 µl of the fixation/permeabilization solution was added, and the cells were incubated on ice in darkness for 2 h. Then, 1 ml of the fixation/permeabilization wash buffer was added, the cells were centrifuged at 500 g for 5 minutes, and the supernatant was carefully removed. Cells were resuspended in the fixation/permeabilization wash buffer, and intracellular antibodies Foxp3 and IL-17A were added at a ratio of 1:200. The cells were incubated on ice in darkness for 30 minutes. After washing with 1 ml of the FACS buffer and centrifugation at 500 g for 5 minutes, the supernatant was carefully removed. Cells were resuspended in 200 µl of the FACS buffer and prepared for flow cytometry analysis.

### 1.2.6 Subcutaneous xenograft tumor model in mice

All animal experiments were approved by the Animal Ethics Committee of the Institute of Nutrition Sciences, Shanghai Institutes for Biological Sciences, Chinese Academy of Sciences, and were conducted in accordance with the protocols approved by the Animal Care and Use Committee of Shanghai Institutes for Biological Sciences. Six-week-old C57BL/6J mice were purchased from Shanghai Slake Experimental Animal Co., Ltd. and were housed in an SPF-grade animal facility. For the subcutaneous tumorigenesis experiment, cells were digested, centrifuged, collected, and resuspended in PBS, and then 2×10⁵ NSCLC cells were injected intraperitoneally into each mouse. The tumor size was measured every two days, and the tumor volume (mm³) was calculated using the following formula: 1/2×a×b², wherein a was the long diameter and b was the short diameter of the tumor. After the mice were euthanized, the tumors were excised, weighed, and the average weight of tumors in each group was calculated. The excised tumors were fixed with paraformaldehyde for subsequent immunohistochemical or immunofluorescence analysis.

### 1.2.7 RNA-seq analysis

RNA from mouse tumor tissues was extracted as previously described. Sample quality inspection and sequencing services were provided by Shanghai Majorbio Bio-pharm Technology Co., Ltd. After fragmentation, reverse transcription, and ligation with adaptors, samples were sequenced on the Illumina NovaSeq6000 platform. Raw data were filtered using the fastp software (https://github.com/OpenGene/fastp) to obtain high-quality sequencing data (clean reads) after sequencing. Clean reads were mapped to the mouse genome (GRCm38.p5) using HISAT2. Differential gene expression analysis between groups was performed using the DESeq2 software. GO and KEGG pathway analyses were conducted using R packages, with significantly differentially expressed genes (P < 0.05) as target genes. GO enrichment analysis was performed using the Goatools software (https://github.com/tanghaibao/GOatools), and KEGG pathway enrichment analysis was conducted using the KOBAS (http://kobas.cbi.pku.edu.cn/home.do). Data mining and graphical presentation, including KEGG, GSEA, heatmaps, and clustering, etc., were all performed on the Majorbio Cloud Platform (https://cloud.majorbio.com).

### 1.2.8 Data statistics

All experiments were repeated at least three times, and *in vivo* experiments were repeated at least twice. Unpaired two-tailed Student's t-test was used to detect significance between cell groups or different mouse groups. According to the test results, a P-value between 0.01 and 0.05 was designated as "*", a P-value between 0.001 and 0.05 was designated as "**", and a P-value less than 0.001 was designated as "***". Quantitative statistics of Western Blot was performed using Image J, and immunofluorescence was analyzed using Image J or Photoshop CS6. Graphs were generated using Graph Pad Prism 8.0.

### Experimental results

C57BL/6J mice were subcutaneously injected with LLC cells (2*10^5 cells per mouse) to establish xenograft tumors. After 14 days of tumor formation, mice received SeMet treatment for two weeks (Figure 1A). The tumor growth curves in mice were plotted, and it was found that SeMet treatment can effectively inhibit the growth of subcutaneous LLC tumors (Figure 1B-C).

RNA-Seq deep sequencing was conducted on fresh tumor samples obtained from *in vivo* experiments. Through comparing with the three major databases of KEGG/Reactome/GO, significant changes were identified in signal transduction and the immune system following selenium treatment (Figure 2A-D). These results indicate that there are significant changes in positive immune regulation and cytokine stimulation after selenium treatment, suggesting that the tumor immune microenvironment has been positively altered after selenium treatment.

We detected multiple genes related to the expression of selenoproteins in tumor tissues and found that compared with the control group, the SeMet-treated group showed a significant increase in the expression of selenoproteins SOD1, SOD2, GPX1, GPX2, GPX4, Sephs2, Sepx1, SelS, SelP, Txnrd1, Txnrd2, and Txnrd3 at the mRNA level in xenograft tumors (Figure 3A-L), and a significant increase in the expression of SOD1 and GPX1 at the protein level (Figure 3M-N), indicating the effectiveness of selenium supplementation.

Through detection, it was found that SeMet treatment increased the expression of PD-L1 in LLC xenograft tumors (Figure 4A-D).

Subsequently, we investigated the therapeutic effects of SeMet and PD-1 antibody used alone or in combination on LLC cell subcutaneous xenograft tumors in mice. Mice were first subcutaneously injected with LLC cells (2*10^5 cells per mouse) to establish xenograft tumors. After 14 days of tumor formation, mice were treated with SeMet and PD-1 antibody alone or in combination by intraperitoneal injection for two weeks. The dosage and frequency of SeMet were the same as previously described, while the dosage of PD-1 antibody was 200 µg per mouse, 2 times per week (Figure 5A). Statistical analysis of tumor growth in mice showed that compared with the control group, tumor growth was significantly reduced in mice treated with SeMet or PD-1 antibody alone; however, the combined treatment did not exhibit a better effect (Figure 5B). The statistical analysis of the ratio of tumor to body weight in mice also validated this result, and visual inspection of excised subcutaneous mouse tumors revealed that the tumors in SeMet-treated mice were significantly reduced (Figure 5C-D).

RNA was extracted from mouse tumors to investigate gene expression levels at the mRNA level. It was found that compared with the control group, SeMet treatment indeed increased the expression of selenoproteins GPX1, GPX2, SOD1, SOD2, Sephs2, Sepx1, SelS, Txnrd1, Txnrd2, SelF, Selk, SelW, and MT2 in LLC xenograft tumors at the mRNA level (Figure 6A-M). SeMet treatment significantly increased the selenium content in subcutaneous xenograft tumors of mice.

Previous RNA-seq analysis of mouse subcutaneous tumors indicated significant changes in immune-related pathways after SeMet treatment (Figure 2A-D). CD4⁺ T cells play a crucial role in regulating immune homeostasis in tumors. A large number of regulatory T cells (Treg) are present in the tumor microenvironment to help tumor cells evade immune surveillance. Therefore, promoting the anti-tumor immune response of CD4⁺ T cells became a focus of our study. The regulation of CD4⁺ T cell-related subsets by SeMet and PD-1 antibody treatment was then explored. First, through qPCR analysis at the mRNA level, we found that SeMet treatment reduced the expression levels of CD4 and Foxp3 in LLC xenograft tumors compared with the control group (Figure 7A). After digesting mouse tumors into single cells, flow cytometry analysis revealed that treatment with SeMet or PD-1 antibody significantly increased the number of CD45⁺ immune cells (Figure 7B-C). Further analysis of CD25⁺Foxp3⁺ Treg cells showed that only SeMet treatment significantly reduced the proportion of Treg cells, while PD-1 antibody treatment had no significant effect on the number of Treg cells, and the combined treatment of SeMet and PD-1 antibody caused a minor reduction in the proportion of Treg cells (Figure 7D-E). These results indicated that SeMet treatment could decrease the number of immunosuppressive Treg cells in LLC cell subcutaneous xenograft tumors and enhance anti-tumor effects. Another differentiation subtype of CD4⁺ T cells, TH17 cells, can inhibit tumor growth and proliferation by secreting related cytokines, anti-tumor angiogenesis, and recruiting and activating CD8⁺ T cells. Therefore, we also tested the effector functions of TH17 cells. The results showed that SeMet treatment significantly increased the proportion of IL-17a-secreting TH17 cells, while PD-1 antibody treatment or the combined treatment of SeMet and PD-1 antibody had no significant effect on the proportion of TH17 cells (Figure 7F-G). These results indicated that SeMet treatment enhanced the anti-tumor function of CD4⁺ T cells and exerted a positive regulatory effect on tumor immunity.

All literatures mentioned in the present invention are incorporated herein by reference, as though each one is individually incorporated by reference. In addition, it should be understood that, after reading the above teachings of the present invention, those skilled in the art can make various changes or modifications to the present invention, these equivalents also fall within the scope as defined in the appended claims of the present application.

## Claims

1. Use of a seleno-amino acid in the manufacture of a composition or formulation for preventing and/or treating a cancer.

2. The use of claim 1, wherein the seleno-amino acid comprises an L-seleno-amino acid.

3. The use of claim 1, wherein the seleno-amino acid comprises a selenomethionine.

4. The use of claim 3, wherein the selenomethionine comprises an L-selenomethionine.

5. The use of claim 1, wherein the cancer comprises lung cancer.

6. A pharmaceutical composition, comprising:
(a1) a first active ingredient for preventing and/or treating a cancer, wherein the first active ingredient comprises: a seleno-amino acid;
(a2) optionally, a second active ingredient for preventing and/or treating a cancer, wherein the second active ingredient comprises: other medicaments for preventing and/or treating a cancer; and
(b) a pharmaceutically acceptable carrier.

7. The pharmaceutical composition of claim 6, wherein the other medicaments for preventing and/or treating a cancer comprise PD-1 antibodies.

8. A pharmaceutical kit comprising:
(i) a first container, and an active ingredient (a1) a seleno-amino acid, or a medicament comprising the active ingredient (a1), contained in the first container; and
(ii) optionally a second container, and an active ingredient (a2), other medicaments for preventing and/or treating a cancer, or a medicament comprising the active ingredient (a2), contained in the second container.

9. Use of the pharmaceutical composition of claim 6 or the pharmaceutical kit of claim 8, in the manufacture of a medicament for preventing and/or treating a cancer.

10. A method for screening candidate medicaments for preventing and/or treating a cancer, comprising the steps of:
(a) in a test group, in the presence of a test substance, culturing cells expressing selenoproteins for a period of time T1 in a culture system, and detecting the expression level E1 and/or activity A1 of selenoproteins in the culture system of the test group;
and in a control group where the test substance is absent and other conditions are the same, detecting the expression level E2 and/or activity A2 of selenoproteins in the culture system of the control group;
(b1) comparing E1 and E2, and if E1 is significantly higher than E2, it indicates that the test substance is a candidate medicament for preventing and/or treating a cancer; and/or
(b2) comparing A1 and A2, and if A1 is significantly higher than A2, it indicates that the test substance is a candidate medicament for preventing and/or treating a cancer.
